(19) Europäisches Patentamt
European Patent Office
Office européen des brevets

(11) Publication number: 0 047 611
B1

(12) **EUROPEAN PATENT SPECIFICATION**

(45) Date of publication of patent specification: **14.11.84**

(21) Application number: **81303921.1**

(22) Date of filing: **27.08.81**

(51) Int. Cl.³: **C 07 D 487/04,** C 12 P 17/18 // C12R1/465 ,(C07D487/04, 209/00, 205/00)

# (54) Process for producing an antibiotic.

(30) Priority: **05.09.80 JP 123087/80**

(43) Date of publication of application: **17.03.82 Bulletin 82/11**

(45) Publication of the grant of the patent: **14.11.84 Bulletin 84/46**

(84) Designated Contracting States: **AT DE FR GB IT NL SE**

(56) References cited:
**FR-A-2 447 922**

**SYNTHETIC METHODS OF ORGANIC CHEMISTRY, vol. 30, 1976 editor: WILLIAM THEILHEIMER** ***abstract no. 656, page 508***

**Organic Functional Group Preparations, Acedemic Press, 1972, p. 115**

**The Condensed Chemical Dictionary, 9th Ed., p. 666**

**The file contains technical information submitted after the application was filed and not included in this specification**

(73) Proprietor: **KOWA COMPANY, LTD.
6-29, 3-chome Nishiki
Naka-ku Nagoya-shi Aichi-ken (JP)**

(72) Inventor: **Nakayama, Masahito
Green Town 2-801 3-1293-10 Tateno
Higashi-Yamato-shi Tokyo (JP)**
Inventor: **Kimura, Shigeru
4-51-1, Kashiwa-cho Tachikawa-shi
Tokyo (JP)**
Inventor: **Mizoguchi, Toshimi
2-17-43, Noguchi-cho Higashi-Murayama-shi
Tokyo (JP)**
Inventor: **Tanabe, Sohei
2-17-43, Noguchi-cho Higashi-Murayama-shi
Tokyo (JP)**
Inventor: **Mori, Toshihito
4-16-3, Fujimi-cho, Higashi-Murayama-shi
Tokyo (JP)**

(74) Representative: **Webb, Frederick Ronald et al
G.F. Redfern & Co. Marlborough Lodge 14
Farncombe Road
Worthing West Sussex BN11 2BT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a novel process for producing an antibiotic KA—6643—A, or a salt thereof.

The present applicants have found that a new strain KC—6643 belonging to the genus *Streptomyces* can be used to prepare a novel antibiotic possessing potent antibacterial activity against gram-positive and gram-negative bacteria. This new strain was deposited with the Fermentation Research Institute of the Agency of Industrial Science & Technology located at No. 1—3, Higashi 1-chome, Yatabe-machi, Tsukuba-gun, Ibaragi-ken, Japan, on April 7, 1978, and has been given Acceptance No. FERM—P 4467. The strain was also deposited with the American Type Culture Collection located at No. 12301 Parklawn Drive, Rockville, Maryland, the United States of America, on March 12, 1979, and has been given ATCC Acceptance No. ATCC Acceptance No. ATCC 31493. As a result of this finding, they have succeeded in isolating two new antibiotics, KA—6643—A and KA—6643—B, from culture broths of this strain, as disclosed in published U.K. Patent Application No. 2,047,700 A in the name of the present Applicants.

In general, these novel antibiotics are represented by the formula (III):

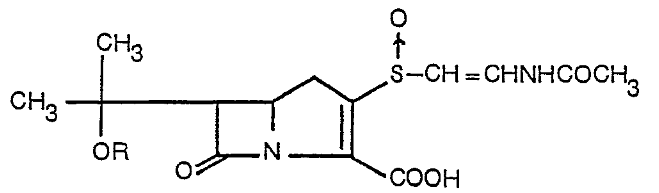

(III)

The formula (III) represents the antibiotic KA—6643—A when R is a hydrogen atom and the antibiotic KA—6643—B when R is a sulfonic acid group. Both the antibiotic KA—6643—A (hereinafter referred to as "KA—6643—A") and the antibiotic KA—6643—B (hereinafter referred to as "KA—6643—B") exhibit marked antibacterial potency. It has been found that the former is superior in potency to the latter.

In various studies leading to the present invention, it has now been found that KA—6643—A can easily be obtained from KA—6643—B by hydrolysis.

It is, therefore, an object of the present invention to provide a novel technique for producing KA—6643—A of excellent antibacterial characteristics with operationally and economically satisfactory results.

According to the invention, there is provided a process for producing KA—6643—A, represented by the formula (I):

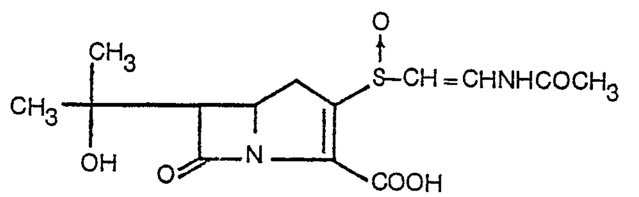

(I)

or a salt thereof, which comprises hydrolyzing KA—6643—B, represented by the formula (II):

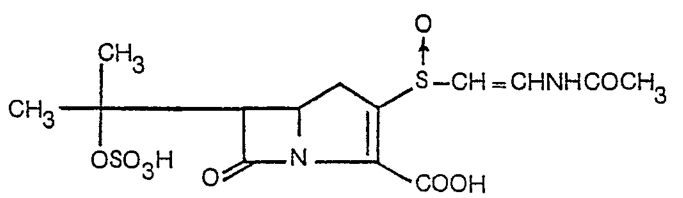

(II)

or a salt thereof, the hydrolysis being carried out in an aqueous phosphate or acetate buffer solution at a pH of 6 to 7.

The KA—6643 substances exist in two isomeric forms, i.e., *cis* and *trans*-forms, because the substances contain a double bond in their side chain. This *cis*-isomer is represented as the Z-form and has the formula (IV):

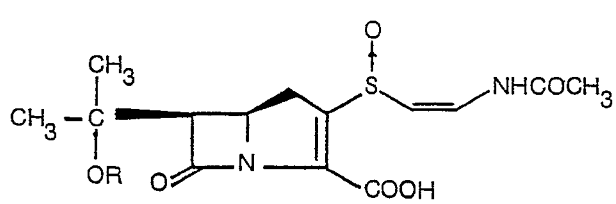

(IV)

wherein R is a hydrogen atom or a sulfonic acid group, and the *trans*-isomer is represented as the E-form and has the formula (V):

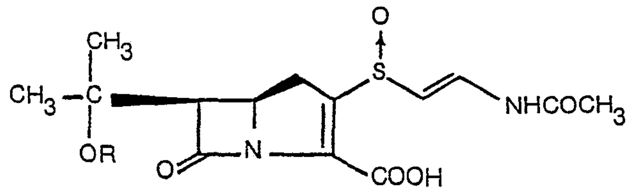

(V)

wherein R is the same as before.

As a metabolic product, the product obtained is the E-form. When reacted with a mercuric salt, however, the E-form is converted to the Z form, as described in Japanese Patent Application No. 55—86989. Accordingly, it is possible to utilize either of the E- and Z-forms as a starting material for the process of the present invention. Instead of isolating the KA—6643 substance, either from a filtrate of a culture broth of a strain belonging to the genus *Streptomyces* and capable of preparing the substance, or from a crude active fraction obtained by culturing the strain, KA—6643—B can also be converted to KA—6643—A by hydrolyzing such a culture broth filtrate or crude active fraction.

For carrying out the process according to the invention, the hydrolysis reaction is effected using an aqueous phosphate or acetate buffer solution at a pH of 6 to 7.

An anti-oxidant such as acidic sodium sulfite, may, if desired, be added to the hydrolysis reaction system.

The hydrolysis reaction is suitably carried out at temperatures of from room temperature to 100°C, for periods of time at from 1 to 10 hours.

The isolation of the desired product (KA—6643—A) from the reaction mixture and the subsequent purification of the product may be effected by any commonly used technique, preferably by gel filtration, column chromatography, reversed phase chromatography, or freeze-drying, or any combination thereof.

If preferred, the KA—6643—A may be prepared as or converted to an alkali metal salt; an alkaline earth metal salt; a primary, secondary or tertiary amine salt; a quaternary ammonium salt; or to a lower alkyl ester or fatty acid ester thereof.

The invention is illustrated by the following Reference Example and non-limitative Examples 1 and 2.

Reference Example

(i) Into a medium which had a composition of 2% by weight of starch, 1.5% by weight of soybean flour, 0.28% by weight of monobasic potassium phosphate, 0.18% by weight of dibasic sodium phosphate dodecahydrate, 0.005% by weight of cobalt chloride hexahydrate, 0.05% by weight of magnesium sulfate heptahydrate and 0.001% by weight of ferrous sulfate and which had been adjusted to a pH of 6.0 and sterilized were inoculated mycelia of a KC—6643 strain, followed by pre-culture at 27°C for about 48 hours to give a first seed culture. Into each of two tanks, each having a volume of 200 l, there was charged 100 l of another medium of the same composition as used above, but having 1% by weight of cotton seed oil added. The first seed culture was inoculated into each tank in an amount of 500 ml and cultured at 30°C for 4 days by an aerated agitation system (rpm: 300, flow rate of air: 50 l/min).

(ii) After completion of the culture, 10 v/v% of Dicalite Perlite 4109 (Dicalite Orient Co., Ltd.) was added to the culture broth (170 l) as a filter aid, and the mycelia were removed by filtration. The electrical conductivity of the resulting filtrate (150 l) was adjusted to 1.5 mΩ/cm and the filtrate was then passed through a column (21.5 × 45 cm) of a strongly basic anion exchange resin, 'Diaion PA316" ($Cl^-$ type: Mitsubishi Chem. Ind., Ltd.) at a flow rate of 200 ml/min. After being washed with a 0.01 M phosphate buffer solution (pH: 7.0), the resin was subjected to elution with a 0.01M phosphate buffer solution (pH: 7.0) containing 2M of sodium chloride in 2 v/v% of aqueous methanol, at a flow rate of 150 ml/min, to collect an active fraction. The resulting active fraction was passed through a column (16 × 10 cm) of "Diaion HP—20", which had been adjusted with a 10 w/v% sodium chloride solution, at a flow rate of 400 ml/min and, after being washed with a 10 w/v% sodium chloride solution, was eluted with deionized water at a flow rate of 200 ml/min to collect an active fraction.

(iii) The active fraction obtained in (ii) above was passed through a column (6 × 70 cm) of a strongly basic anion exchange resin, "Amberlite IRA—458" ($Cl^-$ type: Rohm & Haas Co.), at a flow rate of 50 ml/min and eluted with a 0.01M phosphate buffer solution (pH: 7.0) containing 0.15M sodium chloride to collect a fraction in which KA—6643—A was included. Thereafter the fraction was eluted with a 0.01M phosphate buffer solution (pH: 7.0) containing 2 M sodium chloride to yield an active fraction.

(iv) The active fraction obtained in (iii) above was passed through a column (6 × 70 cm) of "Diaion HP—20" which had been pre-treated with a 20 w/v% aqueous sodium chloride solution, and eluted with deionized water at a flow rate of 20 ml/min to collect an active fraction. The active fraction was diluted with deionized water to have an electrical conductivity of about 700 μΩ/cm and passed through

a column (3 x 30 cm) of a weakly basic anion exchange resin, "DEAE—Sephadex A—25" (Pharmacia Fine Chemicals), which had been adjusted to a pH of 7.0 using a 0.01M phosphate buffer solution, followed by washing with water and by elution with a phosphate buffer solution (pH; 7.0) containing 0.15M of sodium chloride at a flow rate of 35 ml/min to collect an active fraction. The active fraction was concentrated under reduced pressure at below 30°C, passed through a column (3.5 x 40 cm) of "Diaion HP—20" wich had been pre-treated with a 10 w/v% aqueous sodium chloride solution, and then eluted with deionized water at a flow rate of 20 ml/min to collect an active fraction. The resulting active fraction was concentrated under reduced pressure to about 2 ml and freeze-dried to yield 190 mg of a crude powder of KA—6643—B. The crude powder thus obtained was dissolved in 1 ml of deionized water, passed through a column (2 x 30 cm) of "Diaion HP—20" and thereafter eluted with deionized water at a flow rate of 1 ml/min to collect an active fraction. The active fraction was concentrated under reduced pressure and passed through a column of "Sephadex G—10", followed by development with deionized water at a flow rate of 2.0 ml/min. The resulting active fraction was collected, concentrated under reduced pressure to about 2 ml and then freeze-dried to yield 80 mg of a crude powder.

(v) 80 mg of the crude powder obtained in (iv) above was dissolved in 0.1 ml of a 0.1M phosphate buffer solution (pH: 6.8) and passed through a column (0.8 x 120 cm) of "Bondapack $C_{18}$"/"Polasil B" (Waters Associates, Inc.) which had been pre-treated with a 0.1M phosphate buffer solution, followed by elution with the same buffer solution at a flow rate of 6 ml/min to collect an active fraction. The active fraction was passed through a column (0.9 x 5 cm) of active carbon, washed with deionized water and then eluted with 50 w/v% aqueous acetone to collect an active fraction. The resulting active fraction was concentrated under reduced pressure to about 1 ml and freeze-dried to yield 35 mg of a pure powder of KA—6643—B.

Example 1

80 mg of a sodium salt of KA—6643—B was dissolved in 2 ml of 0.1M phosphate buffer solution (pH: 7.0), and the solution was allowed to stand in a water bath at 70°C for 4 hours. After being cooled to room temperature, the solution was passed through a column (8 x 1,200 mm) of "Bondapack $C_{18}$" for adsorption. Elution was performed at a flow rate of 6 ml/min by means of a 0.05 M phosphate buffer solution (pH: 6.8) containing 3% methanol. KA—6643—B was eluted after a retention time of 35 minutes, whereas KA—6643—A was eluted after a retention time of 120 minutes. The fraction containing KA—6643—A was collected and concentrated to about 5 ml under reduced pressure. The concentrate was passed through a column (20 x 200 mm) of "Diaion HP—20" which had been pre-treated with a 10% sodium chloride solution and eluted with deionized water. The active fraction was collected and concentrated to about 2 ml. The concentrate was passed through a column (25 x 1,000 mm) of "Sephadex G—10" and eluted with deionized water. The resulting active fraction was collected and freeze-dried to yield 2.2 mg of a sodium salt of KA—6643—A. The ultimate substance was identical with the authentic product derived from fermentation in terms of its physicochemical and biological properties.

Example 2

60 mg of a sodium salt of KA—6643—B was dissolved in 10 ml of 0.1M acetate buffer solution (pH: 6.0), and the solution was allowed to stand in a water bath at 60°C for 2.5 hours. After being cooled to room temperature, the solution was passed through a column (20 x 150 mm) of "QAE—Sephadex A—25". Elution was performed by a concentration gradient method using a 0—0.9% sodium chloride solution. The active fraction was collected and concentrated to about 3 ml under reduced pressure. The concentrate was passed through a column (20 x 200 ml) of "Diaion HP—20" pre-treated with a 10% sodium chloride solution and eluted with deionized water. The active fraction was collected and concentrated to about 2 ml under reduced pressure. The concentrate was passed through a column (25 x 1,000 mm) of "Sephadex G—10" and eluted with deionized water. The resulting active fraction was collected and freeze-dried to yield 1.8 mg of a sodium salt of KA—6643—A.

("DICALITE", "DIAION", "AMBERLITE", "SEPHADEX", "BONDAPACK" and "POLASIL" are Trade Marks).

**Claims**

1. A process for producing an antibiotic KA—6643—A represented by the formula (I):

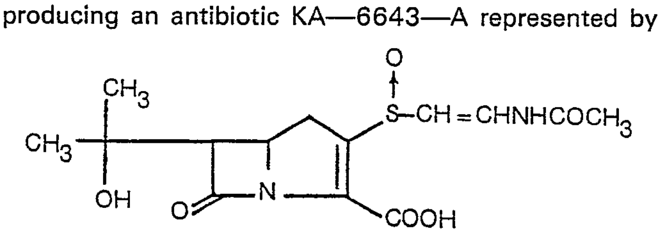

or a salt thereof, which comprises hydrolyzing an antibiotic KA—6643—B represented by the formula (II):

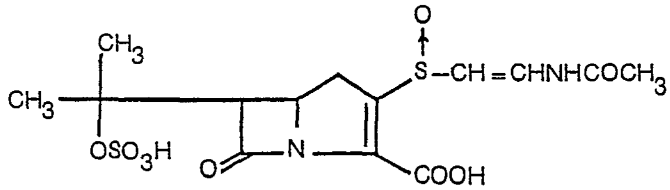

or a salt thereof, the hydrolysis being carried out in an aqueous phosphate or acetate buffer solution at a pH of 6 to 7.

2. A process as claimed in Claim 1, wherein said antibiotic KA—6643—B is isolated from a culture broth of a strain belonging to the genus *Streptomyces* and which is capable of forming a KA—6643 substance.

3. A process as claimed in Claim 2, wherein a crude active fraction obtained by culturing said strain is hydrolyzed.

**Revendications**

1. Procédé pour la production d'un antibiotique KA—6643—A représenté par la formule (I):

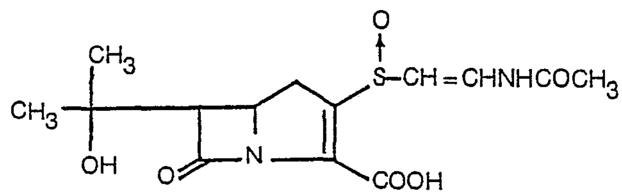

ou d'un sel de celui-ci, qui comprend l'hydrolyse d'un antibiotique KA—6643—B représenté par la formule (II):

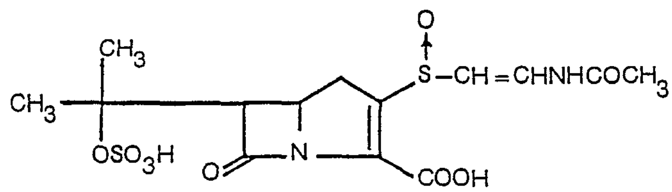

ou d'un sel de celui-ci, l'hydrolyse étant effectuée dans une solution aqueuse de tampon phosphate ou acétate à un pH de 6 à 7.

2. Procédé selon la revendication 1, dans lequel ledit antibiotique KA—6643—B est isolé d'un bouillon de culture d'une souche appartenant au genre *Streptomyces* et qui est capable de former une substance KA—6643.

3. Procédé selon la revendication 2, dans lequel on hydrolyse une fraction active brute obtenue par culture de ladite souche.

**Patentansprüche**

1. Verfahren zur Herstellung eines Antibiotikums KA—6643—A mit der Formel (I):

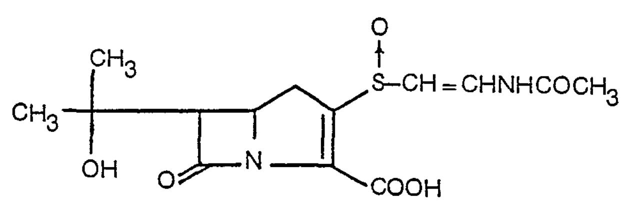

oder eines Salzes desselben, dadurch gekennzeichnet, daß man ein Antibiotikum KA—6643—B mit der allgemeinen Formel (II):

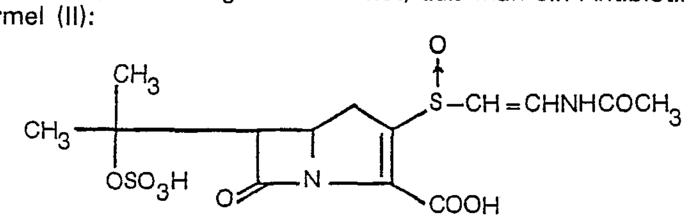

oder ein Salz desselben hydrolysiert, wobei die Hydrolyse in einer wässerigen Phosphat- oder Acetatpufferlösung bei einem pH von 6 bis 7 durchgeführt wird.

2. Verfahren nach Anspruch 1, dadurch gekennzeichnet, daß das Antibiotikum KA—6643—B aus einer Kulturbrühe eines Stammes der Gattung Streptomyces, welcher zur Bildung der Substanz KA—6643 befähigt ist, isolisert wird.

3. Verfahren nach Anspruch 2, dadurch gekennzeichnet, daß man eine durch Kultivieren dieses Stammes erhaltene rohe aktive Fraktion hydrolysiert.